# EUROPEAN PATENT SPECIFICATION

(11) **EP 4 236 872 B1**
(45) Date of publication and mention of the grant of the patent: **04.12.2024**
(21) Application number: 21801645.9
(22) Date of filing: 24.10.2021
(51) Int. Cl.: A61F 2/24

(54) **ATRIOVENTRICULAR VALVE FRAME WITH OPPOSING SETS OF ARMS**
ATRIOVENTRIKULÄRER KLAPPENRAHMEN MIT GEGENÜBERLIEGENDEN ARMSÄTZEN
CADRE DE VALVE ATRIOVENTRICULAIRE AVEC ENSEMBLES DE BRAS OPPOSÉS

(30) Priority: 27.10.2020 US 202063106034 P
(43) Date of publication of application: 06.09.2023
(73) Proprietor: Tel HaShomer Medical Research Infrastructure and Services Ltd., 5265601 Ramat-Gan (IL)
(72) Inventor: ORLOV, Boris, 7564038 Rishon Letzion (IL)
(74) Representative: Evens, Paul Jonathan
(86) International application number: PCT/IB2021/059799
(87) International publication number: WO 2022/090882

(56) References cited:
- EP-A2- 2 852 354
- WO-A1-2020/100050
- US-A1- 2011 137 410
- US-A1- 2014 222 136

## Description

### CROSS-REFERENCES TO RELATED APPLICATIONS

The present application claims priority from US Provisional Patent Application 63/106,034 to Orlov, filed Oct. 27, 2020, entitled "Atrioventricular valve frame with opposing sets of arms".

### FIELD OF EMBODIMENTS OF THE INVENTION

The present invention relates to medical apparatus, and specifically to apparatus for implanting a prosthetic valve at an atrioventricular valve.

### BACKGROUND

The human heart is a muscular organ that pumps deoxygenated blood through the lungs to oxygenate the blood and pumps oxygenated blood to the rest of the body by contractions of four chambers.

After having circulated in the body, deoxygenated blood from the body enters the right atrium through the vena cava(s). In a healthy subject, the right atrium contracts, pumping the blood through the tricuspid valve into the right ventricle. The right ventricle contracts, pumping the blood through the pulmonary semi-lunar valve into the pulmonary artery which splits to two branches, one for each lung. The blood is oxygenated while passing through the lungs, and reenters the heart via the left atrium. The left atrium contracts, pumping the oxygenated blood through the mitral valve into the left ventricle. The left ventricle contracts, pumping the oxygenated blood through the aortic valve into the aorta to be distributed to the rest of the body. The tricuspid valve closes during right ventricle contraction, so that backflow of blood into the right atrium is prevented. Similarly, the mitral valve closes during left ventricle contraction, so that backflow of blood into the left atrium is prevented. The mitral valve and the tricuspid valve are known as atrioventricular valves, each of these valves controlling the flow of blood between an atrium and a ventricle.

In the mitral valve, the mitral annulus defines a mitral valve orifice. An anterior leaflet and a posterior leaflet extend from the mitral annulus. The leaflets are connected by chords to papillary muscles within the left ventricle.

During ventricular diastole, in a healthy subject, the left atrium contracts to pump blood into the left ventricle through the mitral valve orifice. The blood flows through the orifice, pushing the leaflets apart and into the left ventricle with little resistance. In a healthy subject, the leaflets of the aortic valve are kept closed by blood pressure in the aorta.

During ventricular systole, the left ventricle contracts to pump blood into the aorta through the aortic valve, the leaflets of which are pushed open by the blood flow. In a healthy subject, the mitral annulus contracts, pushing the leaflets inwards and reducing the area of the mitral valve orifice by about 20% to 30%. The leaflets coapt to accommodate the excess leaflet surface area, producing a coaptation surface that constitutes a seal. The pressure of blood in the left ventricle pushes against the ventricular surfaces of the leaflets, tightly pressing the leaflets together at the coaptation surface so that a tight, leak-proof seal is formed.

An effective seal of the mitral valve during ventricular systole depends on a sufficient degree of coaptation. Improper coaptation may be caused by any number of physical anomalies that allow leaflet prolapse (for example, elongated or ruptured chords, or weak papillary muscles) or prevent coaptation (for example, short chords, or small leaflets). There are also pathologies that lead to a mitral valve insufficiency, including collagen vascular disease, ischemic mitral regurgitation (resulting, for example, from myocardial infarction, chronic heart failure, or failed/unsuccessful surgical or catheter revascularization), myxomatous degeneration of the leaflets, and rheumatic heart disease. Mitral valve regurgitation leads to many complications including arrhythmia, atrial fibrillation, cardiac palpitations, chest pain, congestive heart failure, fainting, fatigue, low cardiac output, orthopnea, paroxysmal nocturnal dyspnea, pulmonary edema, shortness of breath, and sudden death.

The tricuspid valve includes three leaflets: the septal leaflet, the anterior leaflet, and the posterior leaflet. Each of the valve leaflets is attached to the tricuspid valve annulus, which defines the tricuspid valve orifice. The leaflets are connected to papillary muscles within the right ventricle, by chords. In a healthy subject the tricuspid valve controls the direction of blood flow from the right atrium to the right ventricular, in a similar manner to the control of the mitral valve over the direction of blood flow on the left side of the heart. During ventricular diastole, the tricuspid valve opens, such as to allow the flow of blood from the right atrium to the right ventricle, and during ventricular systole the leaflets of the tricuspid valve coapt, such as to prevent the backflow of blood from the right ventricle to the right atrium.

Tricuspid valve regurgitation occurs when the tricuspid valve fails to close properly. This can cause blood to flow back up into the right atrium when the right ventricle contracts. Tricuspid valve regurgitation is most commonly caused by right ventricle dilation, which leads to the tricuspid valve annulus dilating, resulting in the valve leaflets failing to coapt properly

US2014/222136A1 discloses an example of an apparatus for use with a prosthetic valve.

### SUMMARY OF EMBODIMENTS

In accordance with some applications of the present invention, a prosthetic mitral valve frame includes a valve-frame body that defines a ventricular portion (which upon deployment is configured to be disposed within the subject's left ventricle), and an atrial portion (which upon deployment is configured to be disposed within the subject's left atrium). A prosthetic mitral valve, which typically includes a plurality of leaflets (e.g., two leaflets, or three leaflets, as shown), is typically sutured or otherwise coupled to the valve-frame body. Typically, in a non-constrained configuration of the prosthetic mitral valve frame, the first and second sets of chord-recruiting arms extend radially from a portion of the valve-frame body that is configured to be placed within the subject's ventricle. For some applications, the chord-recruiting arms are configured to extend axially from a ventricular end of the valve-frame body (i.e., the end of the valve-frame body that is configured to be placed within the ventricle) toward an atrial end of the valve-frame body (i.e., the end of the valve-frame body that is configured to be placed within the atrium). Typically, each of the first and second sets of chord-recruiting arms curves around the outside of the valve-frame body in a respective, different circumferential direction of curvature. For example, the first set may curve in the counterclockwise direction and the second set in the clockwise direction, or vice versa. For some applications, the arms belonging to the first set of chord-recruiting arms are configured to have concavely rounded leading edges facing in the first circumferential direction, and the arms belonging to the second set of chord-recruiting arms are configured to have concavely rounded leading edges facing in the second circumferential direction.

Typically, the prosthetic mitral valve and the prosthetic mitral valve frame are delivered to the native mitral valve, using a delivery catheter, and the delivery catheter is configured to maintain the prosthetic mitral valve and prosthetic mitral valve frame in radially-constrained configurations (i.e., "crimped" configurations) during the delivery. When the distal end of the delivery catheter is disposed within the subject's left ventricle, the first set of the chord-recruiting arms are allowed to assume non-radially-constrained configurations and at least partially radially expand. Subsequent to the first set of chord-recruiting arms being deployed among chords of the native mitral valve (and, typically, while the chord-recruiting arms belonging to the other set of chord-recruiting arms are maintained in radially-constrained configurations by the delivery catheter), at least a portion of the valve frame is rotated in the same circumferential direction as the direction of the circumferential curvature of the chord-recruiting arms belonging to the first set. The rotation of the first set of chord-recruiting arms is such as to cause the first set of chord-recruiting arms to (a) pull the native mitral valve radially inward toward the valve frame, and (b) twist the native mitral valve around the valve frame, by recruiting and deflecting at least a portion of the chords.

Typically, either prior or subsequent to the above-described first rotation step being performed, the second set of chord-recruiting arms are allowed to assume non-radially-constrained configurations and at least partially radially expand. Subsequent to the first rotation step having been performed, the valve frame is rotated in the same circumferential direction as the direction of the circumferential curvature of the second set of the chord-recruiting arms. Typically, the rotation of the valve frame in this manner causes chords of the native mitral valve to become entangled between the two sets of chord-recruiting arms, which strengthens the anchoring of the prosthetic mitral valve frame to the native mitral valve apparatus, relative to if the prosthetic mitral valve frame only included a single set of chord-recruiting arms that curve in a single circumferential direction. Typically, the angle through which the valve frame is rotated in the second rotation step is less than or equal to the angle through which the valve frame is rotated in the second rotation step, in order to prevent chords from tearing.

Subsequent to both sets of chord-recruiting arms having been released and the valve frame having been rotated in first and second circumferential directions, the valve-frame body is allowed to assume its non-radially-constrained configurations. Typically, by the valve-frame body assuming its non-radially-constrained configurations, the valve-frame body is configured to trap the native valve leaflets in a partially closed and twisted configuration, to thereby at least partially seal a space between the native mitral valve and the prosthetic mitral valve.

The term "distal" and related terms, when used with reference to a device or a portion thereof, should be interpreted to mean an end of the device or the portion thereof that, when inserted into a subject's body, is typically further from the location through which the device is inserted into the subject's body. The term "proximal" and related terms, when used with reference to a device or a portion thereof, should be interpreted to mean an end of the device or the portion thereof that, when inserted into a subject's body, is typically closer to the location through which the device is inserted into the subject's body.

There is therefore provided, in accordance with the present invention, apparatus for use with a prosthetic valve that is configured to be deployed within a native atrioventricular valve of a heart of a mammalian subject, the native atrioventricular valve including a valve annulus, valve leaflets, chords, and papillary muscles, the apparatus comprising:
a valve frame, the valve frame comprising:
a valve-frame body that is configured to support the prosthetic valve within the native atrioventricular valve;
a first set of chord-recruiting arms extending from the valve-frame body configured to curve around the valve-frame body circumferentially in a first circumferential direction; and
a second set of chord-recruiting arms extending from the valve-frame body and configured to curve around the valve-frame body circumferentially in a second circumferential direction that is an opposite direction from the first circumferential direction.

In some applications, the first set of chord-recruiting arms are configured to extend radially from the valve-frame body.

In some applications, the first set of chord-recruiting arms are configured to extend axially from a ventricular end of the valve-frame body to an atrial end of the valve frame body.

In some applications, the second set of chord-recruiting arms are configured to extend radially from the valve-frame body.

In some applications, the second set of chord-recruiting arms are configured to extend axially from a ventricular end of the valve-frame body to an atrial end of the valve frame body.

According to the present invention, the valve frame is configured such that rotating the valve frame in the first circumferential direction causes the first set of chord-recruiting arms to (a) pull the native atrioventricular valve radially inward toward the valve frame, and (b) twist the native atrioventricular valve around the valve frame, by recruiting and deflecting at least a portion of the chords of the native atrioventricular valve.

According to the present invention, the valve frame is configured such that rotating the valve frame in the second circumferential direction subsequent to the valve frame being rotated in the first direction causes cause the chords to become entangled between the first and second sets of chord-recruiting arms.

In some applications, the valve-frame body is configured to radially expand, such as to trap the leaflets of the native atrioventricular valve in a partially closed and twisted configuration, to thereby at least partially seal a space between the native atrioventricular valve and the prosthetic valve.

There is further provided a method for use with a prosthetic valve that is configured to be deployed within a native atrioventricular valve of a heart of a mammalian subject, the native atrioventricular valve including a valve annulus, valve leaflets, chords, and papillary muscles, the method including:
placing a valve frame within the subject's heart, the valve frame including a valve-frame body that is configured to support the prosthetic valve within the native atrioventricular valve, and at least first and second sets of chord-recruiting arms that are configured to extend from the valve-frame body;
deploying the first set of chord-recruiting arms, such that the first set of chord-recruiting arms become deployed among chords of the native atrioventricular valve, and the first set of chord-recruiting arms curve around the valve-frame body circumferentially in a first circumferential direction;
rotating at least a portion of the valve frame, in the first circumferential direction, such as to cause the first set of chord-recruiting arms to (a) pull the native atrioventricular valve radially inward toward the valve frame, and (b) twist the native atrioventricular valve around the valve frame, by recruiting and deflecting at least a portion of the chords of the native atrioventricular valve;
deploying the second set of chord-recruiting arms, such that the second set of chord-recruiting arms become deployed among chords of the native atrioventricular valve, and the second set of chord-recruiting arms curve around the valve-frame body circumferentially in a second circumferential direction that is an opposite direction from the first circumferential direction; and
rotating at least a portion of the valve frame, in the second circumferential direction, such as to cause the chords to become entangled between the first and second sets of chord-recruiting arms.

In some applications, the method further includes causing the valve-frame body to radially expand, such as to trap the leaflets of the native atrioventricular valve in a partially closed and twisted configuration, to thereby at least partially seal a space between the native atrioventricular valve and the prosthetic valve.

In some applications, deploying the first set of chord-recruiting arms such that the first set of chord-recruiting arms become deployed among chords of the native atrioventricular valve includes deploying the first set of chord-recruiting arms such that the first set of chord-recruiting arms extend radially from the valve-frame body.

In some applications, deploying the first set of chord-recruiting arms such that the first set of chord-recruiting arms become deployed among chords of the native atrioventricular valve includes deploying the first set of chord-recruiting arms such that the first set of chord-recruiting arms extend axially from a ventricular end of the valve-frame body to an atrial end of the valve frame body.

In some applications, deploying the second set of chord-recruiting arms such that the second set of chord-recruiting arms become deployed among chords of the native atrioventricular valve includes deploying the second set of chord-recruiting arms such that the second set of chord-recruiting arms extend radially from the valve-frame body.

In some applications, deploying the first set of chord-recruiting arms such that the second set of chord-recruiting arms become deployed among chords of the native atrioventricular valve includes deploying the second set of chord-recruiting arms such that the second set of chord-recruiting arms extend axially from a ventricular end of the valve-frame body to an atrial end of the valve frame body.

In some applications, deploying the second set of chord-recruiting arms such that the second set of chord-recruiting arms become deployed among chords of the native atrioventricular valve includes deploying the second set of chord-recruiting arms such that the second set of chord-recruiting arms become deployed among chords of the native atrioventricular valve subsequent to rotating the portion of the valve frame in the first circumferential direction.

In some applications, deploying the second set of chord-recruiting arms such that the second set of chord-recruiting arms become deployed among chords of the native atrioventricular valve includes deploying the second set of chord-recruiting arms such that the second set of chord-recruiting arms become deployed among chords of the native atrioventricular valve prior to rotating the portion of the valve frame in the first circumferential direction.

In some applications, rotating at least the portion of the valve frame in the second circumferential direction includes rotating the portion of the valve frame in the second circumferential direction through an angle that is less than an angle through which the portion of the valve frame is rotated during the rotation of the portion of the valve frame in the first circumferential direction.

In some applications, rotating at least the portion of the valve frame in the second circumferential direction includes rotating the portion of the valve frame in the second circumferential direction through an angle that is equal to an angle through which the portion of the valve frame is rotated during the rotation of the portion of the valve frame in the first circumferential direction.

There is further provided, in accordance with some applications of the present invention, apparatus for use with a prosthetic valve that is configured to be deployed within a native atrioventricular valve of a heart of a mammalian subject, the native atrioventricular valve including a valve annulus, valve leaflets, chords, and papillary muscles, the apparatus including:
a valve frame, the valve frame including a valve-frame body that is configured to support the prosthetic valve within the native atrioventricular valve, and at least first and second sets of chord-recruiting arms that are configured to extend from the valve-frame body; and
a delivery device configured to:
   deliver the valve frame to the native atrioventricular valve;
   deploy the first set of chord-recruiting arms, such that the first set of chord-recruiting arms become deployed among chords of the native atrioventricular valve, and the first set of chord-recruiting arms curve around the valve-frame body circumferentially in a first circumferential direction;
   rotate at least a portion of the valve frame, in the first circumferential direction, such as to cause the arm to (a) pull the native atrioventricular valve radially inward toward the valve frame, and (b) twist the native atrioventricular valve around the valve frame, by recruiting and deflecting at least a portion of the chords of the native atrioventricular valve;
   deploy the second set of chord-recruiting arms, such that the second set of chord-recruiting arms become deployed among chords of the native atrioventricular valve, and the second set of chord-recruiting arms curve around the valve-frame body circumferentially in a second circumferential direction that is an opposite direction from the first circumferential direction; and
   rotating at least a portion of the valve frame, in the second circumferential direction, such as to cause the chords to become entangled between the first and second sets of chord-recruiting arms.

In some applications, the delivery device is configured to cause the valve-frame body to radially expand, such as to trap the leaflets of the native atrioventricular valve in a partially closed and twisted configuration, to thereby at least partially seal a space between the native atrioventricular valve and the prosthetic valve.

In some applications, the first set of chord-recruiting arms are configured to extend radially from the valve-frame body.

In some applications, the first set of chord-recruiting arms are configured to extend axially from a ventricular end of the valve-frame body to an atrial end of the valve frame body.

In some applications, the second set of chord-recruiting arms are configured to extend radially from the valve-frame body.

In some applications, the second set of chord-recruiting arms are configured to extend axially from a ventricular end of the valve-frame body to an atrial end of the valve frame body.

In some applications, the delivery device is configured to deploy the second set of chord-recruiting arms such that the second set of chord-recruiting arms become deployed among chords of the native atrioventricular valve, subsequent to rotating the portion of the valve frame in the first circumferential direction.

In some applications, the delivery device is configured to deploy the second set of chord-recruiting arms such that the second set of chord-recruiting arms become deployed among chords of the native atrioventricular valve, prior to rotating the portion of the valve frame in the first circumferential direction.

In some applications, the delivery device is configured to rotate at least the portion of the valve frame in the second circumferential direction through an angle that is less than an angle through which the portion of the valve frame is rotated during the rotation of the portion of the valve frame in the first circumferential direction.

In some applications, the delivery device is configured to rotate at least the portion of the valve frame in the second circumferential direction through an angle that is equal to an angle through which the portion of the valve frame is rotated during the rotation of the portion of the valve frame in the first circumferential direction.

The present invention will be more fully understood from the following detailed description of applications thereof, taken together with the drawings, in which:

### BRIEF DESCRIPTION OF THE DRAWINGS

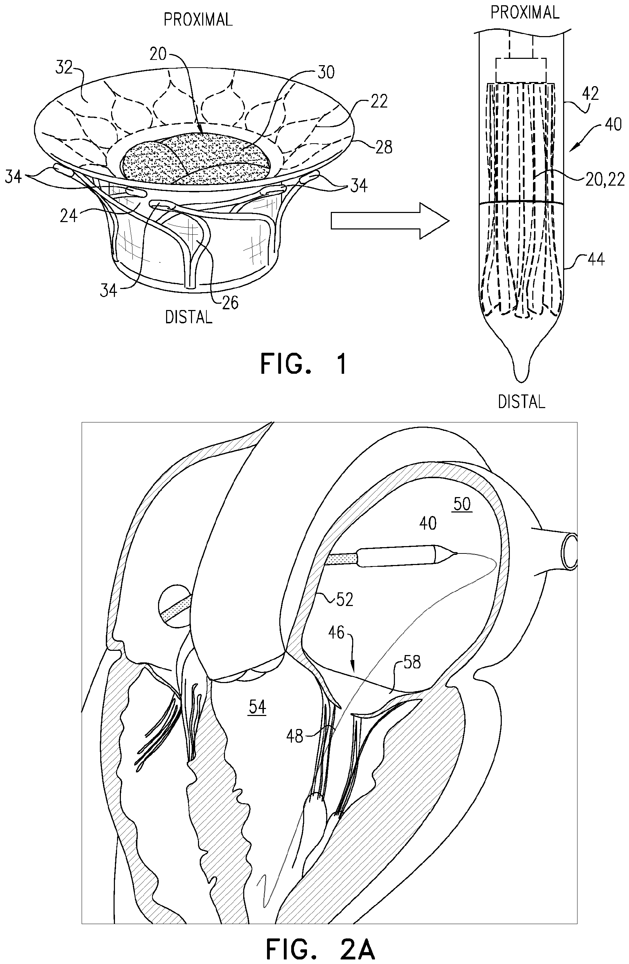

Fig. 1 is a schematic illustration of a prosthetic mitral valve and a prosthetic mitral valve frame, in accordance with some applications of the present invention;

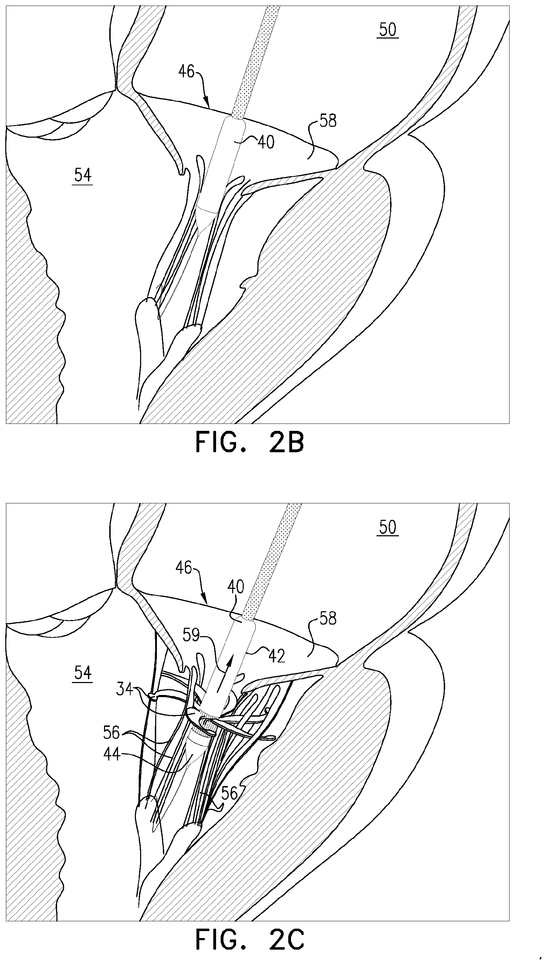

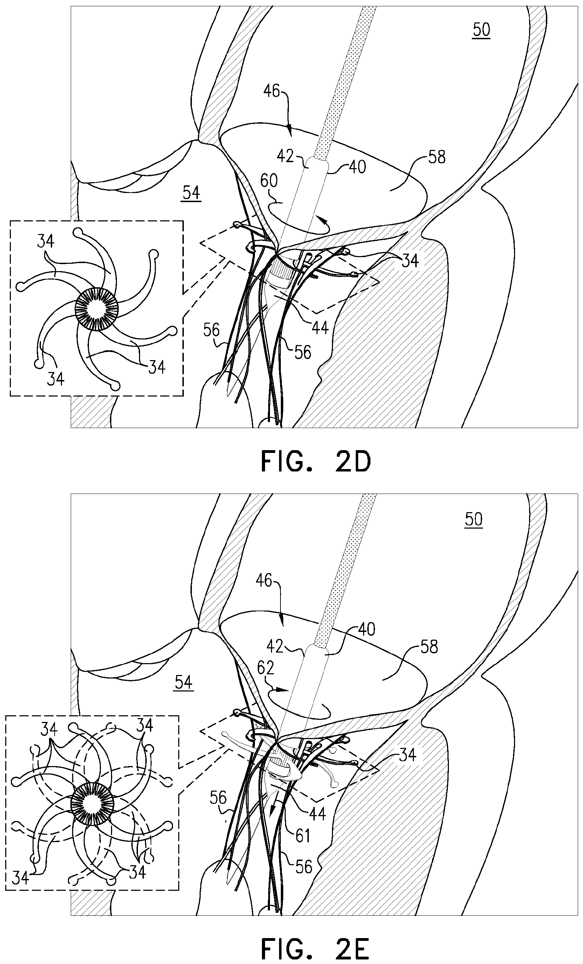

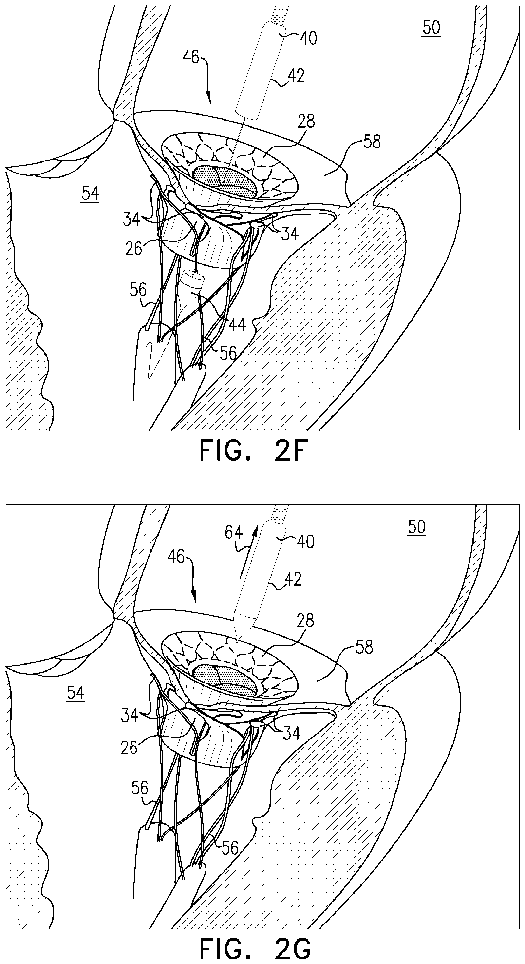

Figs. 2A, 2B, 2C, 2D, 2E, 2F, and 2G are schematic illustrations of respective steps of the deployment of the prosthetic mitral valve and the prosthetic mitral valve frame at a subject's native mitral valve, via a transseptal delivery approach, in accordance with some applications of the present invention; and

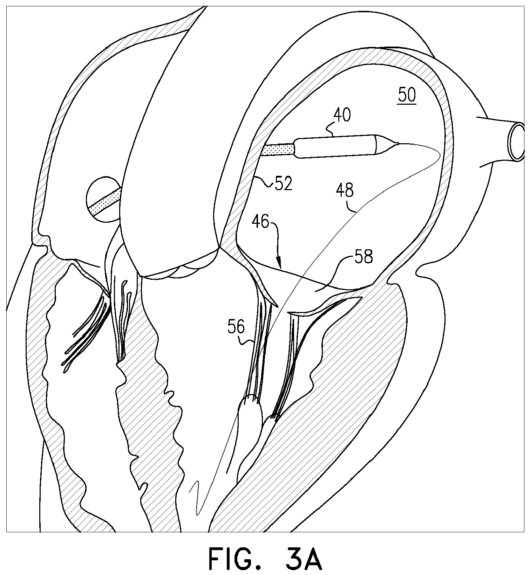

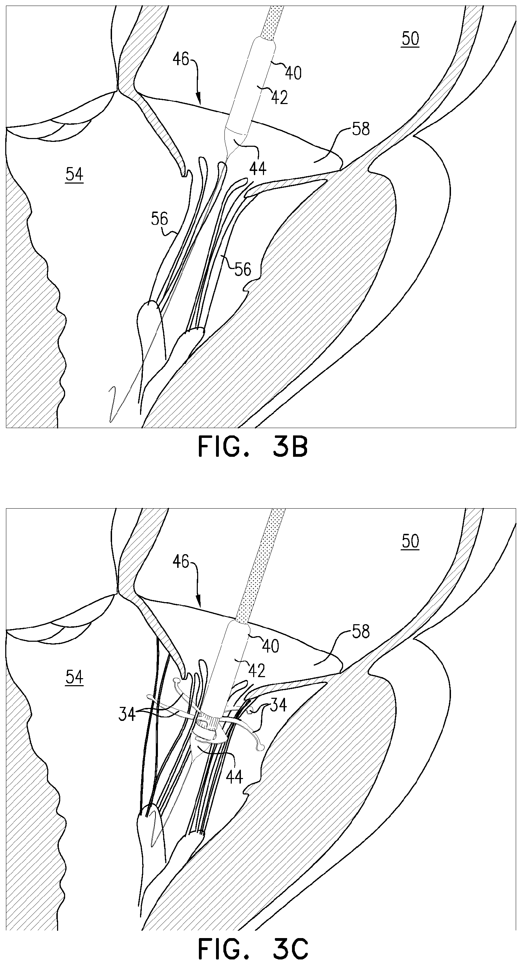

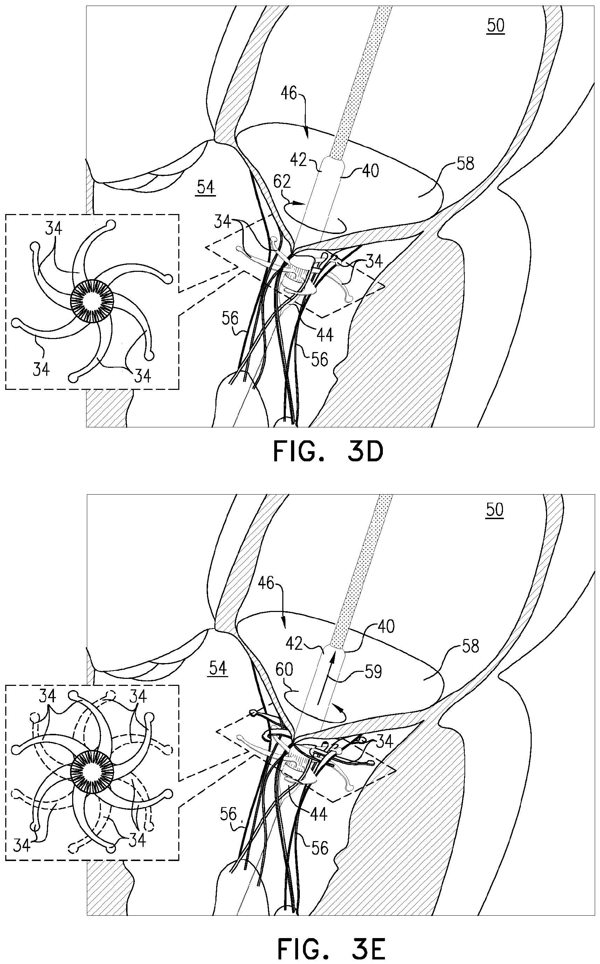

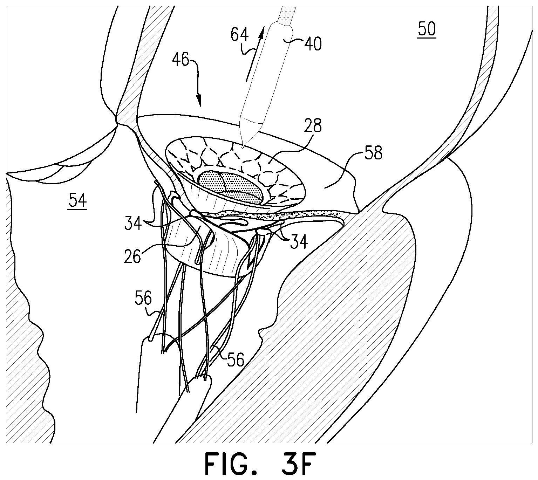

Figs. 3A, 3B, 3C, 3D, 3E, and 3F are schematic illustrations of respective steps of the deployment of the prosthetic mitral valve and the prosthetic mitral valve frame at a subject's native mitral valve, via a transseptal delivery approach, in accordance with some alternative applications of the present invention.

### DETAILED DESCRIPTION OF EMBODIMENTS

Reference is now made to Fig. 1, which is a schematic illustration of a prosthetic mitral valve 20 and a prosthetic mitral valve frame 22, in accordance with some applications of the present invention. The prosthetic mitral valve frame includes a valve-frame body 24 that defines ventricular portion 26 (which upon deployment is configured to be disposed within the subject's left ventricle), and an atrial portion 28 (which upon deployment is configured to be disposed within the subject's left atrium). Prosthetic mitral valve 20 typically includes a plurality of leaflets 30 (e.g., two leaflets, or three leaflets, as shown), which are sutured or otherwise coupled to the valve-frame body.

Typically, valve frame 22 is made of a shape-memory material (e.g., a shape-memory alloy, such as nitinol and/or copper-aluminum-nickel), which is covered on one or both sides with a covering material 32, e.g., a fabric and/or a polymer (such as expanded polytetrafluoroethylene (ePTFE), or woven, knitted and/or braided polyester). Typically, the shape-memory material of valve frame is shaped into a stent-like structure that comprises struts and/or cells of the shape-memory material. The covering material is typically coupled to the shape-memory material via stitches.

Typically, in a non-constrained configuration of prosthetic mitral valve frame 22, first and second sets of chord-recruiting arms 34 extend radially from a portion of valve-frame body 24 that is configured to be placed within the subject's ventricle. For some applications, each of the sets of chord-recruiting arms includes a plurality of chord-recruiting arms, for example, more than 2 (e.g., more than 4), and/or fewer than 15 (e.g., fewer than 8), e.g., 2-15 or 4-8 chord-recruiting arms. Typically, the chord-recruiting arms are configured to extend radially from the valve-frame body, in addition to extending axially from a ventricular end of the valve-frame body (i.e., the end of the valve-frame body that is configured to be placed within the ventricle) toward an atrial end of the valve-frame body (i.e., the end of the valve-frame body that is configured to be placed within the atrium). Each of the first and second sets of chord-recruiting arms curves around the outside of the valve-frame body in a respective, different circumferential direction of curvature. For example, the first set may curve in the counterclockwise direction and the second set in the clockwise direction, or vice versa. For some applications, the arms belonging to the first set of chord-recruiting arms are configured to have concavely rounded leading edges facing in the first circumferential direction, and the arms belonging to the second set of chord-recruiting arms are configured to have concavely rounded leading edges facing in the second circumferential direction.

Typically, prosthetic mitral valve 20 and prosthetic mitral valve frame 22 are delivered to the native mitral valve, using a delivery catheter 40 (shown in the right portion of Fig. 1, for example), and the delivery catheter is configured to maintain prosthetic mitral valve 20 and prosthetic mitral valve frame 22 in radially-constrained configurations (i.e., "crimped" configurations) during the delivery. For some applications, the delivery catheter includes a proximal covering sheath 42 (which is configured to maintain a proximal portion of the prosthetic mitral valve frame in a radially-constrained configuration by covering the proximal portion), and a distal nose cone 44 (which is configured to maintain a distal portion of the prosthetic mitral valve frame in a radially-constrained configuration by covering the distal portion).

Reference is now made to Figs. 2A, 2B, 2C, 2D, 2E, 2F, and 2G, which are schematic illustrations of respective steps of the deployment of prosthetic mitral valve 20 and prosthetic mitral valve frame 22 at a subject's native mitral valve 46, via a transseptal delivery approach, in accordance with some applications of the present invention. Typically, the delivery catheter is guided toward the subject's native mitral valve 46 over a guidewire 48. In accordance with respective applications, the prosthetic mitral valve 20 and prosthetic mitral valve frame 22 are delivered transseptally (i.e., via the vena cava, the right atrium, and the interatrial septum), transapically (i.e., via the apex of the left ventricle), and/or via a different delivery path. Although some aspects of the present application relate to delivery via a transseptal approach, the scope of the present invention includes delivering the prosthetic mitral valve 20 and prosthetic mitral valve frame 22 via a different approach, *mutatis mutandis.*

As shown in Fig. 2A, the distal end of delivery catheter 40 is typically advanced into the subject's left atrium 50, via the interatrial septum 52. The distal end of the delivery catheter is advanced toward the native mitral valve, and is advanced through leaflets 58 of the native mitral valve and into left ventricle 54, as shown in Fig. 2B. When the distal end of the delivery catheter is disposed within the left ventricle, a first set of the chord-recruiting arms 34 are allowed to assume non-radially-constrained configurations and at least partially radially expand, as shown in Fig. 2C. For example, as shown, the chord-recruiting arms belonging to the proximal set of chord-recruiting arms are allowed to assume non-radially-constrained configurations and at least partially radially expand. For some applications, the arms are allowed to assume non-radially-constrained configurations by releasing the arms from being radially constrained by the delivery catheter, e.g., by partially retracting proximal covering sheath 42, e.g., as indicated by arrow 59 in Fig. 2C, and/or by partially advancing distal nose cone 44. Typically, the chord-recruiting arms belonging to the proximal set are shape set to extend radially from valve-frame body 24 and to curve circumferentially around the valve-frame body (e.g., in the counterclockwise direction, as shown), upon being released from being radially constrained by the delivery catheter. Further typically, the chord-recruiting arms are configured to become deployed among chords 56 of the native mitral valve upon being released from being radially constrained by the delivery catheter. For some applications, at this stage, the chord-recruiting arms belonging to the second set of chord-recruiting arms are maintained in radially-constrained configurations by delivery catheter 40 (e.g., distal nose cone 44 of the delivery catheter), as shown in Fig. 2C. Alternatively, the chord-recruiting arms belonging to the second set of chord-recruiting arms are also allowed to assume their non-radially-constrained configurations, e.g., by advancing distal nose cone 44 of the delivery catheter, and/or by retracting proximal covering sheath 42.

As shown in Fig. 2D, subsequent to the first set of chord-recruiting arms 34 being deployed among chords of the native mitral valve (and typically while the chord-recruiting arms belonging to the other set of chord-recruiting arms are maintained in radially-constrained configurations by delivery catheter 40), at least a portion of valve frame 22 is rotated in the direction of arrow 60, such as to cause chord-recruiting arms 34 to (a) pull the native mitral valve radially inward toward the valve frame, and (b) twist the native mitral valve around the valve frame, by recruiting and deflecting at least a portion of the chords. Typically, the first set of chord-recruiting arms 34 are configured to curve in a given circumferential direction with respect to the longitudinal axis of the valve frame. For example, the arms may curve in a clockwise direction or in a counterclockwise direction with respect to the longitudinal axis of the valve frame. Typically, subsequent to chord-recruiting arms 34 being deployed among chords of the native mitral valve, the valve frame is rotated in the same circumferential direction as the direction of the circumferential curvature of the arms. In the example shown in Fig. 2D, the first set of arms curve in the counterclockwise circumferential direction (as viewed from left atrium 50), and the valve frame is rotated in this direction. As noted above, for some applications, the chord-recruiting arms belonging to the second set of chord-recruiting arms are allowed to assume their non-radially-constrained configurations, prior to the rotation step that is shown in Fig. 2D being performed. For such applications, during the rotation step that is illustrated in Fig. 2D, the chords typically slide over the outer edges of the chord-recruiting arms belonging to the second set.

As shown in Fig. 2E, for some applications, only subsequent to the rotation of the valve-frame, the second set of the chord-recruiting arms 34 are allowed to assume non-radially-constrained configurations and at least partially radially expand. For example, as shown, the chord-recruiting arms belonging to the distal set of chord-recruiting arms (which is the second set of chord-recruiting arms, in the example being shown) are allowed to assume non-radially-constrained configurations and at least partially radially expand. For some applications, the arms are allowed to assume non-radially-constrained configurations by releasing the arms from being radially constrained by the delivery catheter, e.g., by partially advancing distal nose cone 44 as indicated by arrow 61 of Fig. 2E. Typically, the chord-recruiting arms belonging to the second set are shape set to extend radially from valve-frame body 24 and to curve circumferentially around the valve-frame body, upon being released from being radially constrained by the delivery catheter. Further typically, the chord-recruiting arms are configured to become deployed among chords 56 of the native mitral valve upon being released from being radially constrained by the delivery catheter.

As described hereinabove, the chord-recruiting arms belonging to the distal set are typically shape set such that the circumferential curvature of the chord-recruiting arms belonging to the distal set is in the opposite circumferential direction from that of the chord-recruiting arms belonging to the proximal set. For example, as shown in Fig. 2E, the arms belonging to the proximal set curve in the counterclockwise direction, while those belonging to the distal set curve in the clockwise direction (as viewed from left atrium 50). As shown in Fig. 2E, subsequent to the first rotation step having been performed, the valve frame is counter-rotated in the direction of the circumferential curvature of the second set of the chord-recruiting arms. In the example shown in Fig. 2E, the second set of arms curve in the clockwise circumferential direction (as viewed from left atrium 50), and the valve frame is rotated in this direction, in the direction of arrow 62. The rotation of the valve frame in this manner causes chords 56 of the native mitral valve to become entangled between the two sets of chord-recruiting arms, which strengthens the anchoring of the prosthetic mitral valve frame to the native mitral valve apparatus, relative to if the prosthetic mitral valve frame only included a single set of chord-recruiting arms that curve in a single circumferential direction. Typically, the angle through which the valve frame is rotated in the second rotation step is less than or equal to the angle through which the valve frame is rotated in the first rotation step, in order to prevent chords 56 from tearing.

Subsequent to both sets of chord-recruiting arms having been released and the valve frame having been rotated in first and second circumferential directions, the valve-frame body (i.e., ventricular portion 26 and atrial portion 28 of the valve frame) is allowed to assume its non-radially-constrained configurations. For some applications, the atrial portion is allowed to assume its non-radially-constrained configuration by releasing the atrial portion from the delivery catheter, e.g., by further retracting proximal covering sheath 42. For some applications, the ventricular portion is allowed to assume its non-radially-constrained configuration by releasing the ventricular portion from the delivery catheter, e.g., by further advancing distal nose cone 44. Fig. 2F shows both ventricular portion 26 and atrial portion 28 in their non-radially-constrained (i.e., radially-expanded) configurations. Typically, by the valve-frame body assuming its non-radially-constrained configuration, the valve-frame body is configured to trap the native valve leaflets 58 in a partially closed and twisted configuration, to thereby at least partially seal a space between the native mitral valve and the prosthetic valve. For example, the ventricular portion may be configured to radially expand such as to trap the native valve leaflets between the ventricular portion and the chord-recruiting arms, and/or the atrial portion may be configured to radially expand such as to trap the native valve leaflets between the atrial portion and the chord-recruiting arms. Further typically, the valve frame is anchored to the native mitral valve apparatus by chords 56 being entangled between arms belonging to the first and second sets of chord-recruiting arms. Subsequent to the above described steps being performed, delivery catheter 40 is typically then retracted in its entirety from the subject's left atrium, as indicated by arrow 64 in Fig. 2G.

Reference is now made to Figs. 3A, 3B, 3C, 3D, 3E, and 3F, which are schematic illustrations of respective steps of the deployment of prosthetic mitral valve 20 and prosthetic mitral valve frame 22 at a subject's native mitral valve 46, via a transseptal delivery approach, in accordance with some applications of the present invention. In general, the procedure described with reference to Figs. 3A-3F is similar to that described with reference to Figs. 2A-2G, except that the order in which the proximal and distal sets of chord-recruiting arms are allowed to assume their non-radially constrained configurations, and the order of the corresponding rotations of the valve frame are reversed, as described in further detail hereinbelow. Thus, while in the example shown in Figs. 2A-G, the proximal set of chord-recruiting arms is considered the first set of chord-recruiting arms (and the distal set of chord-recruiting arms is considered the second set of chord-recruiting arms), in the example shown in Figs. 3A-F, the distal set of chord-recruiting arms is considered the first set of chord-recruiting arms (and the proximal set of chord-recruiting arms is considered the second set of chord-recruiting arms).

Typically, prosthetic mitral valve 20 and prosthetic mitral valve frame 22 are delivered to the native mitral valve, using a delivery catheter 40 (shown in Fig. 3A, for example), and the delivery catheter is configured to maintain prosthetic mitral valve 20 and prosthetic mitral valve frame 22 in radially-constrained configurations (i.e., "crimped" configurations) during the delivery. Typically, the delivery catheter is guided toward a native mitral valve 46 of the subject over a guidewire 48. As shown in Fig. 3A, the distal end of delivery catheter 40 is typically advanced into the subject's left atrium 50, via the interatrial septum 52. The distal end of the delivery catheter is advanced toward the native mitral valve, as shown in Fig. 3B, and is then advanced through leaflets 58 of the native mitral valve and into left ventricle 54.

When the distal end of the delivery catheter is disposed within the left ventricle, a first set of the chord-recruiting arms 34 are allowed to assume non-radially-constrained configurations and at least partially radially expand, as shown in Fig. 3C. For example, as shown, the chord-recruiting arms belonging to the distal set of chord-recruiting arms are allowed to assume non-radially-constrained configurations and at least partially radially expand. For some applications, the arms are allowed to assume non-radially-constrained configurations by releasing the arms from being radially constrained by the delivery catheter, e.g., by partially advancing distal nose cone 44, and/or by partially retracting proximal covering sheath 42. Typically, the chord-recruiting arms belonging to the distal set are shape set to extend radially from valve-frame body 24 and to curve circumferentially around the valve-frame body (e.g., in the clockwise direction, as shown), upon being released from being radially constrained by the delivery catheter. Further typically, the chord-recruiting arms are configured to become deployed among chords 56 of the native mitral valve upon being released from being radially constrained by the delivery catheter. For some applications, at this stage, the chord-recruiting arms belonging to the other set (i.e., the proximal set, in this case) of chord-recruiting arms are maintained in radially-constrained configurations by delivery catheter 40 (e.g., proximal covering sheath 42 of the delivery catheter), as shown in Fig. 3C. Alternatively, the chord-recruiting arms belonging to the second set of chord-recruiting arms are also allowed to assume their non-radially-constrained configurations, e.g., by retracting proximal covering sheath 42 of the delivery catheter, and/or by advancing distal nose cone 44.

As shown in Fig. 3D, subsequent to the first set of chord-recruiting arms 34 being deployed among chords of the native mitral valve (and typically while the chord-recruiting arms belonging to the other set of chord-recruiting arms are maintained in radially-constrained configurations by delivery catheter 40), at least a portion of valve frame 22 is rotated in the direction of arrow 62, such as to cause chord-recruiting arms 34 to (a) pull the native mitral valve radially inward toward the valve frame, and (b) twist the native mitral valve around the valve frame, by recruiting and deflecting at least a portion of the chords. Typically, the first set of chord-recruiting arms 34 are configured to curve in a given circumferential direction with respect to the longitudinal axis of the valve frame. For example, the arms may curve in a clockwise direction or in a counterclockwise direction with respect to the longitudinal axis of the valve frame. Typically, subsequent to chord-recruiting arms 34 being deployed among chords of the native mitral valve, the valve frame is rotated in the same circumferential direction as the direction of the circumferential curvature of the arms. In the example shown in Fig. 3D, the distal set of arms curve in the clockwise circumferential direction (as viewed from left atrium 50), and the valve frame is rotated in this direction. As noted above, for some applications, the chord-recruiting arms belonging to the second set of chord-recruiting arms are allowed to assume their non-radially-constrained configurations, prior to the rotation step that is shown in Fig. 3D being performed. For such applications, during the rotation step that is illustrated in Fig. 3D, the chords typically slide over the outer edges of the chord-recruiting arms belonging to the second set of chord-recruiting arms.

As shown in Fig. 3E, for some applications, only subsequent to the rotation of the valve-frame, the second set of the chord-recruiting arms 34 are allowed to assume non-radially-constrained configurations and at least partially radially expand. For example, as shown, the chord-recruiting arms belonging to the proximal set of chord-recruiting arms (which is the second set of chord-recruiting arms, in the example being shown) are allowed to assume non-radially-constrained configurations and at least partially radially expand. For some applications, the arms are allowed to assume non-radially-constrained configurations by releasing the arms from being radially constrained by the delivery catheter, e.g., by partially retracting proximal covering sheath 42. Typically, the chord-recruiting arms belonging to the proximal set are shape set to extend radially from valve-frame body 24 and to curve circumferentially around the valve-frame body, upon being released from being radially constrained by the delivery catheter. Further typically, the chord-recruiting arms are configured to become deployed among chords 56 of the native mitral valve upon being released from being radially constrained by the delivery catheter.

As described hereinabove, the chord-recruiting arms belonging to the proximal set are typically shape set such that the circumferential curvature of the chord-recruiting arms belonging to the proximal set is in the opposite circumferential direction from that of the chord-recruiting arms belonging to the distal set. For example, as shown in Fig. 3E, the arms belonging to the distal set curve in the clockwise direction, while those belonging to the proximal set curve in the counterclockwise direction. As shown in Fig. 3E, subsequent to the first rotation step having been performed, the valve frame is counter-rotated in the direction of the circumferential curvature of the proximal set of the chord-recruiting arms. In the example shown in Fig. 3E, the proximal set of arms curve in the counterclockwise circumferential direction (as viewed from left atrium 50), and the valve frame is rotated in this direction, in the direction of arrow 60. Typically, the rotation of the valve frame in this manner causes chords 56 of the native mitral valve to become entangled between the two sets of chord-recruiting arms, which strengthens the anchoring of the prosthetic mitral valve frame to the native mitral valve apparatus, relative to if the prosthetic mitral valve frame only included a single set of chord-recruiting arms that curve in a single circumferential direction. Typically, the angle through which the valve frame is rotated in the second rotation step is less than or equal to the angle through which the valve frame is rotated in the first rotation step, in order to prevent chords from tearing.

Subsequent to both sets of chord-recruiting arms having been released and the valve frame having been rotated in first and second circumferential directions, the valve-frame body (i.e., ventricular portion 26 and atrial portion 28 of the valve frame) is allowed to assume its non-radially-constrained configuration. For some applications, the atrial portion is allowed to assume its non-radially-constrained configuration by releasing the atrial portion from the delivery catheter, e.g., by further retracting proximal covering sheath 42. For some applications, the ventricular portion is allowed to assume its non-radially-constrained configuration by releasing the ventricular portion from the delivery catheter, e.g., by further advancing distal nose cone 44. Fig. 3F shows both ventricular portion 26 and atrial portion 28 in their non-radially-constrained (i.e., radially expanded) configurations. Typically, by the valve-frame body assuming its non-radially-constrained configuration, the valve-frame body is configured to trap the native valve leaflets 58 in a partially closed and twisted configuration, to thereby at least partially seal a space between the native mitral valve and the prosthetic valve. For example, the ventricular portion may be configured to radially expand such as to trap the native valve leaflets between the ventricular portion and the chord-recruiting arms, and/or the atrial portion may be configured to radially expand such as to trap the native valve leaflets between the atrial portion and the chord-recruiting arms. The valve frame is anchored to the native mitral valve apparatus by chords 56 being entangled between arms belonging to the first and second sets of chord-recruiting arms. Subsequent to the above described steps being performed, delivery catheter 40 is typically then retracted in its entirety from the subject's left atrium, as indicated by arrow 64 in Fig. 3F.

Although in the examples described herein the proximal set of arms curve in the counterclockwise circumferential direction (and the corresponding rotation of the valve frame is in this direction), and the distal set of arms curve in the clockwise circumferential direction (and the corresponding rotation of the valve frame is in this direction), the scope of the present invention includes the proximal set of arms curving in the clockwise circumferential direction (and the corresponding rotation of the valve frame is in this direction), and the distal set of arms curve in the counterclockwise circumferential direction (and the corresponding rotation of the valve frame is in this direction). Similarly, the scope of the present invention includes the first and second sets of arms being disposed at the same height as each other along the valve frame, or overlapping with each other along the valve frame, alternating with each other along the valve frame, and/or other possible configurations.

The scope of the present invention includes a valve frame that is generally as described herein, but having proximal and distal sets of arms both of which curve in the same direction as each other. Typically, the arms and methods of use therewith are generally as described hereinabove, *mutatis mutandis.* For some such applications, each of the sets of arms is configured to be deployed among chords at a respective, different height within the left ventricle. The arms are used to recruit and deflect the shapes of chords in the manner described hereinabove, at respective, different heights within the left ventricle. Alternatively, during a procedure, only one of the sets of arms may be selected to be deployed among chords and to be used to recruit and deflect the shapes of chords, while the other set of arms may be released from the delivery catheter only when the ventricular portion of the valve frame is released (such that the other set of arms does not become deployed among chords). Typically, the selection of which set of arms to be deployed among the chords is performed by a medical professional, based upon anatomical constraints of the particular patient within whom the valve frame is deployed.

Although some applications of the present invention are described as being utilized in conjunction with a prosthetic mitral valve and a prosthetic mitral valve frame, the scope of the present invention includes using generally similar apparatus and techniques with any prosthetic atrioventricular valve and prosthetic atrioventricular valve frame. Thus, the scope of the present invention includes using generally similar apparatus and techniques with a prosthetic tricuspid valve and prosthetic tricuspid valve frame having a generally similar configuration to the prosthetic mitral valve and the prosthetic mitral valve frame described herein, *mutatis mutandis.* For example, a prosthetic tricuspid valve frame that includes a first and second sets of chord-recruiting arms may be delivered to a subject's native tricuspid valve via the subject's right atrium, using delivery catheter 40. Typically, the first and second sets of chord-recruiting arms are shape set such as to extend radially from the valve-frame body, in addition to extending axially from a ventricular end of the valve-frame body (i.e., the end of the valve-frame body that is configured to be placed within the ventricle) toward an atrial end of the valve-frame body (i.e., the end of the valve-frame body that is configured to be placed within the atrium). Each of the first and second sets of chord-recruiting arms curves around the outside of the valve-frame body in a respective, different circumferential direction of curvature. For example, the first set may curve in the counterclockwise direction and the second set in the clockwise direction, or vice versa.

Typically, chord-recruiting arms belonging to the first set of chord-recruiting arms are allowed to deploy among chords of the native tricuspid valve, by assuming their non-radially constrained configurations, and the valve frame is rotated in the same circumferential direction as the direction of circumferential curvature of the first set of chord-recruiting arms. This causes the chord-recruiting arms to (a) pull the native tricuspid valve radially inward toward the valve frame, and (b) twist the native tricuspid valve around the valve frame, by recruiting and deflecting at least a portion of the chords. For some applications, the second set of the chord-recruiting arms are allowed to assume non-radially-constrained configurations and at least partially radially expand, such as to become deployed among chords of the native tricuspid valve. As described hereinabove, the chord-recruiting arms belonging to the second set are typically shape set such that the circumferential curvature of the chord-recruiting arms belonging to the second set is in the opposite circumferential direction from that of the chord-recruiting arms belonging to the first set. Subsequent to the first rotation step having been performed, the valve frame is rotated in the same circumferential direction as the direction of the circumferential curvature of the second set of the chord-recruiting arms. The rotation of the valve frame in this manner causes chords of the native tricuspid valve to become entangled between the two sets of chord-recruiting arms, which strengthens the anchoring of the prosthetic tricuspid valve frame to the native tricuspid valve apparatus, relative to if the prosthetic tricuspid valve frame only included a single set of chord-recruiting arms that curve in a single circumferential direction.

Subsequent to both sets of chord-recruiting arms having been released and the valve frame having been rotated in first and second circumferential directions, the valve-frame body is allowed to assume its non-radially-constrained configuration. Typically, by the valve-frame body assuming its non-radially-constrained configuration, the valve-frame body is configured to trap the native tricuspid valve leaflets in a partially closed and twisted configuration, to thereby at least partially seal a space between the native tricuspid valve and the prosthetic valve. For example, the ventricular portion may be configured to radially expand such as to trap the native valve leaflets between the ventricular portion and the chord-recruiting arms, and/or the atrial portion may be configured to radially expand such as to trap the native valve leaflets between the atrial portion and the chord-recruiting arms. Further typically, the valve frame is anchored to the native tricuspid valve apparatus by chords being entangled between arms belonging to the first and second sets of chord-recruiting arms.

## Claims

1. Apparatus for use with a prosthetic valve that is configured to be deployed within a native atrioventricular valve of a heart of a mammalian subject, the native atrioventricular valve including a valve annulus, valve leaflets, chords, and papillary muscles, the apparatus comprising:
a valve frame (22), the valve frame (22) comprising:
a valve-frame body (24) that is configured to support the prosthetic valve within the native atrioventricular valve;
a first set of chord-recruiting arms (34) extending from the valve-frame body (24) configured to curve around the valve-frame body (24) circumferentially in a first circumferential direction; and
a second set of chord-recruiting arms (34) extending from the valve-frame body (24) and configured to curve around the valve-frame body (24) circumferentially in a second circumferential direction that is an opposite direction from the first circumferential direction, and
wherein the valve frame (22) is configured such that rotating the valve frame (22) in the first circumferential direction causes the first set of chord-recruiting arms (34) to (a) pull the native atrioventricular valve radially inward toward the valve frame (22), and (b) twist the native atrioventricular valve around the valve frame (22), by recruiting and deflecting at least a portion of the chords of the native atrioventricular valve, and
wherein the valve frame (22) is configured such that rotating the valve frame (22) in the second circumferential direction subsequent to the valve frame (22) being rotated in the first direction causes the chords to become entangled between the first and second sets of chord-recruiting arms (34).

2. The apparatus according to claim 1, wherein the first set of chord-recruiting arms (34) are configured to extend radially from the valve-frame body (24).

3. The apparatus according to claim 1, wherein the first set of chord-recruiting arms (34) are configured to extend axially from a ventricular end of the valve-frame body (24) to an atrial end of the valve frame body.

4. The apparatus according to claim 1, wherein the second set of chord-recruiting arms (34) are configured to extend radially from the valve-frame body (24).

5. The apparatus according to claim 1, wherein the second set of chord-recruiting arms (34) are configured to extend axially from a ventricular end of the valve-frame body (24) to an atrial end of the valve frame body.

6. The apparatus according to claim 1, wherein the valve-frame body (24) is configured to radially expand, such as to trap the leaflets of the native atrioventricular valve in a partially closed and twisted configuration, to thereby at least partially seal a space between the native atrioventricular valve and the prosthetic valve.

7. The apparatus according to claim 1, further comprising:
a delivery device (40) configured to:
deliver the valve frame (22) to the native atrioventricular valve;
deploy the first set of chord-recruiting arms (34), such that the first set of chord-recruiting arms (34) become deployed among chords of the native atrioventricular valve, and the first set of chord-recruiting arms (34) curve around the valve-frame body (24) circumferentially in the first circumferential direction;
rotate at least a portion of the valve frame (22), in the first circumferential direction, such as to cause the first set of chord-recruiting arms (34) to (a) pull the native atrioventricular valve radially inward toward the valve frame (22), and (b) twist the native atrioventricular valve around the valve frame (22), by recruiting and deflecting at least a portion of the chords of the native atrioventricular valve;
deploy the second set of chord-recruiting arms (34), such that the second set of chord-recruiting arms (34) become deployed among chords of the native atrioventricular valve, and the second set of chord-recruiting arms (34) curve around the valve-frame body (24) circumferentially in the second circumferential direction that is an opposite direction from the first circumferential direction; and
rotate at least a portion of the valve frame (22), in the second circumferential direction, such as to cause the chords to become entangled between the first and second sets of chord-recruiting arms (34).

8. The apparatus according to claim 7, wherein the delivery device is configured to cause the valve-frame body (24) to radially expand, such as to trap the leaflets of the native atrioventricular valve in a partially closed and twisted configuration, to thereby at least partially seal a space between the native atrioventricular valve and the prosthetic valve.

9. The apparatus according to claim 7 or 8, wherein the delivery device is configured to deploy the second set of chord-recruiting arms (34) such that the second set of chord-recruiting arms (34) become deployed among chords of the native atrioventricular valve, subsequent to rotating the portion of the valve frame (22) in the first circumferential direction.

10. The apparatus according to claim 7 or 8, wherein the delivery device is configured to deploy the second set of chord-recruiting arms (34) such that the second set of chord-recruiting arms (34) become deployed among chords of the native atrioventricular valve, prior to rotating the portion of the valve frame (22) in the first circumferential direction.

11. The apparatus according to claim 7 or 8, wherein the delivery device is configured to rotate at least the portion of the valve frame (22) in the second circumferential direction through an angle that is less than an angle through which the portion of the valve frame (22) is rotated during the rotation of the portion of the valve frame (22) in the first circumferential direction.

12. The apparatus according to claim 7 or 8, wherein the delivery device is configured to rotate at least the portion of the valve frame (22) in the second circumferential direction through an angle that is equal to an angle through which the portion of the valve frame (22) is rotated during the rotation of the portion of the valve frame (22) in the first circumferential direction.

## Patentansprüche

1. Vorrichtung zur Verwendung mit einer Klappenprothese, die konfiguriert ist, um innerhalb einer nativen atrioventrikulären Klappe eines Herzens eines Säugersubjekts eingesetzt zu werden, wobei die native atrioventrikuläre Klappe einen Klappenring, Klappensegel, Sehnen und Papillarmuskeln beinhaltet, wobei die Vorrichtung Folgendes umfasst:
einen Klappenrahmen (22), wobei der Klappenrahmen (22) Folgendes umfasst:
einen Klappenrahmenkörper (24), der konfiguriert ist, um die Klappenprothese innerhalb der nativen atrioventrikulären Klappe zu stützen;
einen ersten Sehneneinstellungsarmsatz (34), der sich von dem Klappenrahmenkörper (24) erstreckt und konfiguriert ist, um sich um den Klappenrahmenkörper (24) umlaufend in einer ersten Umfangsrichtung zu krümmen; und
einen zweiten Sehneneinstellungsarmsatz (34), der sich von dem Klappenrahmenkörper (24) erstreckt und konfiguriert ist, um sich um den Klappenrahmenkörper (24) umlaufend in einer zweiten Umfangsrichtung zu krümmen, die eine entgegengesetzte Richtung zu der ersten Umfangsrichtung ist, und
wobei der Klappenrahmen (22) konfiguriert ist, sodass Drehen des Klappenrahmens (22) in der ersten Umfangsrichtung bewirkt, dass der erste Sehneneinstellungsarmsatz (34) (a) die native atrioventrikuläre Klappe radial nach innen zu dem Klappenrahmen (22) zieht, und (b) die native atrioventrikuläre Klappe um den Klappenrahmen (22) verdreht, indem zumindest ein Abschnitt der Sehnen der nativen atrioventrikulären Klappe eingestellt und abgelenkt wird, und
wobei der Klappenrahmen (22) konfiguriert ist, sodass Drehen des Klappenrahmens (22) in der zweiten Umfangsrichtung anschließend daran, dass der Klappenrahmen (22) in der ersten Richtung gedreht wird, bewirkt, dass sich die Sehnen zwischen dem ersten und dem zweiten Sehneneinstellungsarmsatz (34) verfangen.

2. Vorrichtung nach Anspruch 1, wobei der erste Sehneneinstellungsarmsatz (34) konfiguriert ist, um sich radial von dem Klappenrahmenkörper (24) zu erstrecken.

3. Vorrichtung nach Anspruch 1, wobei der erste Sehneneinstellungsarmsatz (34) konfiguriert ist, um sich axial von einem ventrikulären Ende des Klappenrahmenkörpers (24) zu einem atrialen Ende des Klappenrahmenkörpers zu erstrecken.

4. Vorrichtung nach Anspruch 1, wobei der zweite Sehneneinstellungsarmsatz (34) konfiguriert ist, um sich radial von dem Klappenrahmenkörper (24) zu erstrecken.

5. Vorrichtung nach Anspruch 1, wobei der zweite Sehneneinstellungsarmsatz (34) konfiguriert ist, um sich axial von einem ventrikulären Ende des Klappenrahmenkörpers (24) zu einem atrialen Ende des Klappenrahmenkörpers zu erstrecken.

6. Vorrichtung nach Anspruch 1, wobei der Klappenrahmenkörper (24) konfiguriert ist, um sich radial auszudehnen, um so die Segel der nativen atrioventrikulären Klappe in einer teilweise geschlossenen und verdrehten Konfiguration einzufangen, um dadurch einen Raum zwischen der nativen atrioventrikulären Klappe und der Klappenprothese zumindest teilweise abzudichten.

7. Vorrichtung nach Anspruch 1, ferner umfassend:
ein Abgabegerät (40), das zu Folgendem konfiguriert ist:
Abgeben des Klappenrahmens (22) an die native atrioventrikuläre Klappe;
Einsetzen des ersten Sehneneinstellungsarmsatz (34), sodass der erste Sehneneinstellungsarmsatz (34) zwischen Sehnen der nativen atrioventrikulären Klappe eingesetzt wird und sich der erste Sehneneinstellungsarmsatz (34) um den Klappenrahmenkörper (24) umlaufend in der ersten Umfangsrichtung krümmt;
Drehen von zumindest einem Abschnitt des Klappenrahmens (22) in der ersten Umfangsrichtung, um zu bewirken, dass der erste Sehneneinstellungsarmsatz (34) (a) die native atrioventrikuläre Klappe radial nach innen zu dem Klappenrahmen (22) zieht, und (b) die native atrioventrikuläre Klappe um den Klappenrahmen (22) verdreht, indem zumindest ein Abschnitt der Sehnen der nativen atrioventrikulären Klappe eingestellt und abgelenkt wird;
Einsetzen des zweiten Sehneneinstellungsarmsatzes (34), sodass der zweite Sehneneinstellungsarmsatz (34) zwischen Sehnen der nativen atrioventrikulären Klappe eingesetzt wird und sich der zweite Sehneneinstellungsarmsatz (34) um den Klappenrahmenkörper (24) umlaufend in der zweiten Umfangsrichtung krümmt, die eine entgegengesetzte Richtung zu der ersten Umfangsrichtung ist; und
Drehen von zumindest einem Abschnitt des Klappenrahmens (22) in der zweiten Umfangsrichtung, um zu bewirken, dass sich die Sehnen zwischen dem ersten und dem zweiten Sehneneinstellungsarmsatz (34) verfangen.

8. Vorrichtung nach Anspruch 7, wobei das Abgabegerät konfiguriert ist, um zu bewirken, dass sich der Klappenrahmenkörper (24) radial ausdehnt, um die Segel der nativen atrioventrikulären Klappe in einer teilweise geschlossenen und verdrehten Konfiguration einzufangen, um dadurch einen Raum zwischen der nativen atrioventrikulären Klappe und der Klappenprothese zumindest teilweise abzudichten.

9. Vorrichtung nach Anspruch 7 oder 8, wobei die Abgabevorrichtung konfiguriert ist, um den zweiten Sehneneinstellungsarmsatz (34) einzusetzen, sodass der zweite Sehneneinstellungsarmsatz (34) zwischen Sehnen der nativen atrioventrikulären Klappe eingesetzt wird, anschließend an das Drehen des Abschnittes des Klappenrahmens (22) in der ersten Umfangsrichtung.

10. Vorrichtung nach Anspruch 7 oder 8, wobei das Abgabegerät konfiguriert ist, um den zweiten Sehneneinstellungsarmsatz (34) einzusetzen, sodass der zweite Sehneneinstellungsarmsatz (34) zwischen Sehnen der nativen atrioventrikulären Klappe eingesetzt wird, vor dem Drehen des Abschnittes des Klappenrahmens (22) in der ersten Umfangsrichtung.

11. Vorrichtung nach Anspruch 7 oder 8, wobei das Abgabegerät konfiguriert ist, um zumindest den Abschnitt des Klappenrahmens (22) in der zweiten Umfangsrichtung durch einen Winkel zu drehen, der kleiner als ein Winkel ist, durch den der Abschnitt des Klappenrahmens (22) während der Drehung des Abschnittes des Klappenrahmens (22) in der ersten Umfangsrichtung gedreht wird.

12. Vorrichtung nach Anspruch 7 oder 8, wobei das Abgabegerät konfiguriert ist, um zumindest den Abschnitt des Klappenrahmens (22) in der zweiten Umfangsrichtung durch einen Winkel zu drehen, der gleich einem Winkel ist, durch den der Abschnitt des Klappenrahmens (22) während der Drehung des Abschnittes des Klappenrahmens (22) in der ersten Umfangsrichtung gedreht wird.

## Revendications

1. Appareil destiné à être utilisé avec une valve prothétique qui est conçue pour être déployés à l'intérieur d'une valve atrio-ventriculaire native d'un coeur d'un sujet mammifère, la valve atrio-ventriculaire native comprenant un anneau valvulaire, des feuillets valvulaires, des cordages et des muscles papillaires, l'appareil comprenant :
un cadre de valve (22), le cadre de valve (22) comprenant :
un corps de cadre de valve (24) qui est conçu pour supporter la valve prothétique à l'intérieur de la valve atrio-ventriculaire native ;
un premier ensemble de bras de recrutement de cordages (34) s'étendant à partir du corps de cadre de valve (24) conçus pour se courber autour du corps de cadre de valve (24) de manière circonférentielle dans une premier sens circonférentiel ; et
un second ensemble de bras de recrutement de cordages (34) s'étendant à partir du corps de cadre de valve (24) et conçus pour se courber autour du corps de cadre de valve (24) de manière circonférentielle dans un second sens circonférentiel qui est un sens opposé au premier sens circonférentiel, et
ledit cadre de valve (22) étant conçu de sorte que la rotation du cadre de valve (22) dans le premier sens circonférentiel amène le premier ensemble de bras de recrutement de cordages (34) à (a) tirer la valve atrio-ventriculaire native radialement vers l'intérieur en direction du cadre de valve (22), et (b) torsader la valve atrio-ventriculaire native autour du cadre de valve (22), en recrutant et en faisant dévier au moins une partie des cordages de la valve atrio-ventriculaire native, et
ledit cadre de valve (22) étant conçu de sorte que la rotation du cadre de valve (22) dans le second sens circonférentiel après la rotation du cadre de valve (22) dans le premier sens provoque l'enchevêtrement des cordages entre les premier et second ensembles de bras de recrutement de cordages (34).

2. Appareil selon la revendication 1, ledit premier ensemble de bras de recrutement de cordages (34) étant conçu pour s'étendre radialement à partir du corps de cadre de valve (24).

3. Appareil selon la revendication 1, ledit premier ensemble de bras de recrutement de cordages (34) étant conçu pour s'étendre axialement d'une extrémité ventriculaire du corps de cadre de valve (24) à une extrémité auriculaire du corps de cadre de valve.

4. Appareil selon la revendication 1, ledit second ensemble de bras de recrutement de cordages (34) étant conçu pour s'étendre radialement à partir du corps de cadre de valve (24).

5. Appareil selon la revendication 1, ledit second ensemble de bras de recrutement de cordages (34) étant conçu pour s'étendre axialement d'une extrémité ventriculaire du corps de cadre de valve (24) à une extrémité auriculaire du corps de cadre de valve.

6. Appareil selon la revendication 1, ledit corps de cadre de valve (24) étant conçu pour se dilater radialement, de manière à piéger les feuillets de la valve atrio-ventriculaire native dans une configuration partiellement fermée et torsadée, pour ainsi fermer hermétiquement au moins partiellement un espace entre la valve atrio-ventriculaire native et la valve prothétique.

7. Appareil selon la revendication 1 comprenant en outre :
un dispositif de pose (40) conçu pour :
la pose du cadre de valve (22) sur la valve atrio-ventriculaire native ;
le déploiement du premier ensemble de bras de recrutement de cordages (34), de sorte que le premier ensemble de bras de recrutement de cordages (34) se déploie parmi les cordages de la valve atrio-ventriculaire native, et le premier ensemble de bras de recrutement de cordages (34) se courbe autour du corps de cadre de valve (24) de manière circonférentielle dans le premier sens circonférentiel ;
la rotation d'au moins une partie du cadre de valve (22) dans le premier sens circonférentiel, de manière à amener le premier ensemble de bras de recrutement de cordages (34) à (a) tirer la valve atrio-ventriculaire native radialement vers l'intérieur en direction du cadre de valve (22), et (b) torsader la valve atrio-ventriculaire native autour du cadre de valve (22), par le recrutement et la déviation d'au moins une partie des cordages de la valve atrio-ventriculaire native ;
le déploiement du second ensemble de bras de recrutement de cordages (34), de sorte que le second ensemble de bras de recrutement de cordages (34) se déploie parmi les cordages de la valve atrio-ventriculaire native, et le second ensemble de bras de recrutement de cordages (34) se courbe autour du corps de cadre de valve (24) de manière circonférentielle dans le second sens circonférentiel qui est un sens opposé au premier sens circonférentiel ; et
la rotation d'au moins une partie du cadre de valve (22), dans le second sens circonférentiel, de manière à provoquer l'enchevêtrement des cordages entre les premier et second ensembles de bras de recrutement de cordages (34).

8. Appareil selon la revendication 7, ledit dispositif de pose étant conçu pour amener le corps de cadre de valve (24) à se dilater radialement, de manière à piéger les feuillets de la valve atrio-ventriculaire native dans une configuration partiellement fermée et torsadée, pour ainsi fermer hermétiquement au moins partiellement un espace entre la valve atrio-ventriculaire native et la valve prothétique.

9. Appareil selon la revendication 7 ou 8, ledit dispositif de pose étant conçu pour déployer le second ensemble de bras de recrutement de cordages (34) de sorte que le second ensemble de bras de recrutement de cordages (34) se déploie parmi les cordages de la valve atrio-ventriculaire native, après la rotation de la partie du cadre de valve (22) dans le premier sens circonférentiel.

10. Appareil selon la revendication 7 ou 8, ledit dispositif de pose étant conçu pour déployer le second ensemble de bras de recrutement de cordages (34) de sorte que le second ensemble de bras de recrutement de cordages (34) se déploie parmi les cordages de la valve atrio-ventriculaire native, avant de faire tourner la partie du cadre de valve (22) dans le premier sens circonférentiel.

11. Appareil selon la revendication 7 ou 8, ledit dispositif de pose étant conçu pour faire tourner au moins la partie du cadre de valve (22) dans le second sens circonférentiel selon un angle qui est inférieur à l'angle selon lequel la partie du cadre de valve (22) est tournée pendant la rotation de la partie du cadre de valve (22) dans le premier sens circonférentiel.

12. Appareil selon la revendication 7 ou 8, ledit dispositif de pose étant conçu pour faire tourner au moins la partie du cadre de valve (22) dans le second sens circonférentiel selon un angle qui est égal à l'angle selon lequel la partie du cadre de valve (22) est tournée pendant la rotation de la partie du cadre de valve (22) dans le premier sens circonférentiel.
